Europäisches Patentamt

European Patent Office

Office européen des brevets

(19)

(11) Numéro de publication: **0 197 062**
**B1**

(12) **FASCICULE DE BREVET EUROPEEN**

(45) Date de publication du fascicule du brevet: **09.01.91**

(21) Numéro de dépôt: **85904647.6**

(22) Date de dépôt: **26.09.85**

(86) Numéro de dépôt international:
**PCT/FR85/00264**

(87) Numéro de publication internationale:
**WO 86/02098 10.04.86 Gazette 86/08**

(51) Int. Cl.⁵: **C 12 P 21/00,** A 61 K 39/008,
G 01 N 33/577

(54) ANTICORPS MONOCLONAUX ANTI-LEISHMANIA, HYBRIDOMES SECRETEURS DE CES ANTICORPS, ANTIGENES DE LEISHMANIA RECONNUS PAR CES ANTICORPS, PROCEDE D'OBTENTION ET APPLICATIONS DE CES ANTICORPS MONOCLONAUX ET DE CES ANTIGENES.

(30) Priorité: **01.10.84 FR 8415079**
**11.02.85 FR 8501892**

(43) Date de publication de la demande:
**15.10.86 Bulletin 86/42**

(45) Mention de la délivrance du brevet:
**09.01.91 Bulletin 91/02**

(84) Etats contractants désignés:
**AT BE CH DE FR GB IT LI LU NL SE**

(56) Documents cités:
**WO-A-83/01785**

**The Journal of Immunology, vol. 133, no. 1, Juillet 1984 The American Association of Immunologists, C.L. Jaffe et al.: "Production and characterization of species-specific monoclonal antibodies against Leishmania Donovani for immunodiagnosis" pages 440-447, voir page 441: "Materials and Methods"**

(73) Titulaire: **INSTITUT PASTEUR**
**25-28, rue du Docteur Roux**
**F-75724 Paris Cédex 15 (FR)**
(73) Titulaire: **UNIVERSITE PIERRE ET MARIE CURIE PARIS VI**
**4, place Jussieu**
**F-75230 Paris Cedex 05 (FR)**
(73) Titulaire: **UNIVERSITE DE TECHNOLOGIE DE COMPIEGNE**
**Centre Benjamin Franklin Rue Roger Couttolenc - BP 233**
**F-60206 Compiègne Cédex (FR)**

(72) Inventeur: **MONJOUR, Louis**
**35, rue Broca**
**F-75005 Paris (FR)**
Inventeur: **ROSETO, Alberto**
**34, avenue Guy de Maupassant**
**F-78400 Chatou (FR)**
Inventeur: **BERNEMAN, Armand**
**11, rue Crussol**
**F-75011 Paris (FR)**
Inventeur: **GUILLEMIN-DEBONS, Marie-Claude**
**34, avenue Guy de Maupassant**
**F-78400 Chatou (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

(56) References cited:
The Journal of Immunology, vol. 131, no. 4, Octobre 1983, The American Association of Immunologists, C.L.Jaffe et al: "1. Characterization of antigens associated with stage and species-specific determinants", pages 1487-1993, voir page 1987: "Materials and Methods" - page 1990, colonne de gauche, ligne 5

Chemical Abstracts, vol. 98, 9 Mai 1983, Columbus, Ohio (US) E. Handmann et al.: "Isolation and characterization of infective and noninfective clones of Leishmania tropica", voir page 237, abrégé 157503n

The Journal of Immunology, vol. 131, no. 3, Septembre 1983, The American Association of Immunologists, H.W. Sheppard et al.: "Species cross-reactivity, functional correlates of cell-mediated immunity, and antigen characterization", pages 1496-1503, voir l'article en entier

Nature, vol. 291, no. 5816, Juin 1981 Macmillan Journals Ltd. Chesham, Bucks (GB) D. McMahon Pratt et al.: "Monoclonal antibodies that distinguish between new world species of Leishmania", pages 581-583, voir l'article en entier

(72) Inventeur: DOMURADO, Martine
Rue du Clos-Moise
Jonquières F-60680 Grandfresnoy (FR)
Inventeur: PERIES, Serge
50, quai de Jemmapes
F-75010 Paris (FR)

(74) Mandataire: Gutmann, Ernest et al
S.C. Ernest Gutmann - Yves Plasseraud 67, boulevard Haussmann
F-75008 Paris (FR)

# EP 0 197 062 B1

**Description**

L'invention est relative à de nouveaux anticorps monoclonaux anti-Leishmania et à des hybridomes secréteurs de tels anticorps monoclonaux. Elle concerne plus particulièrement des anticorps monoclonaux susceptibles d'induire une activité protectrice à l'égard d'un hôte sensible à l'infection par les leishmanies et également de reconnaître des polypeptides provenant de ces parasites et qui sont eux-mêmes reconnus par des sérums obtenus à partir d'hôtes infectés par diverses espèces de leishmanies. Elle concerne enfin des antigènes susceptibles d'être reconnus par ces anticorps et l'utilisation de ces antigènes pour la constitution de compositions de vaccins destinées à l'homme ou à l'animal, notamment les chiens.

Les leishmanioses recouvrent un ensemble complexe d'infections qui ont en commun d'être causées par des parasites intracellulaires du genre Leishmania. On peut se reporter au préambule de la demande internationale de brevet PCT/US 82/01678, déposée le 17 novembre 1982 et publiée sous le no. WO83/0175, pour disposer d'une revue générale des différentes formes que peuvent revêtir ces infections et des différentes espèces de Leishmanies qui en sont la cause. Le développement de ces infections tant dans les pays du tiers monde que dans des pays industrialises est d'autant plus préoccupant que l'on ne dispose à l'heure actuelle que le peu de moyens pour combattre les différentes infections causées par ces parasites et pour enrayer leur extension à de nouveaux territoires. L'arsenal thérapeutique est limité et il n'existe actuellement aucune vaccination pouvant aboutir à une prévention efficace sans complications secondaires.

La diversité antigénique des leishmanies a été mise en évidence notamment par l'utilisation des anticorps monoclonaux. On notera à ce sujet, outre la demande internationale de brevet susmentionée les travaux réalisés par D. McMahon Pratt et John R. David, (1981), Nature, Vol. 291, 581—583; de E. Handman et R. E. Hocking (1982), Infections and Immunity, 28—33; de E. Handman, H. M. Jarvis et G. F. Litchell (1984) Parasite Immunology, *6*, 223—233. L'impression d'ensemble qui se dégage de la littérature existante est précisément la grande variété d'anticorps monoclonaux qui ont été fabriqués, ces anticorps monoclonaux devant permettre des identifications désormais précises des différentes espèces et sous-espèces existantes de Leishmanies. Les publications décrivant des effets de protection d'un hôte vivant contre certaines espèces de leishmanies sont beaucoup plus rares. On citera néanmoins les résultats de protection *in vivo* par des anticorps monoclonaux contre les promastigotes de *Leishmania mexicana* qui ont été obtenus par Anderson et Coll. (1983) The Journal of Immunology, *131*, 1616—1618, à l'occasion de la mise en oeuvre d'un test dénommé "Winn assay system". Le principe de ce test est décrit dans un article de H. J. Winn (1960) publié dans le "J. of Immunology", Vol. 84, p. 530.

L'invention procède d'une démarche différente. En effet, elle avait pour but la production d'anticorps monoclonaux (ou de compositions à base de plusieurs types d'anticorps monoclonaux) qui soient capables, d'une part, de reconnaître des déterminants antigéniques communs à des espèces variées de leishmanies, d'autre part, d'entraîner un effet protecteur *in vivo* contre ces diverses espèces. Un autre objectif était l'isolement d'un ou plusieurs antigènes susceptibles d'induire chez l'hôte auxquels ils peuvent être administrés l'acquisition d'une immunité globale, par conséquent également une protection globale contre les différentes leishmanioses cutanées, cutanéomuqueuses et viscérales de l'Ancien et du Nouveau monde.

Plus particulièrement encore, l'invention a pour but de fournir un procédé permettant de conduire la sélection des hybridomes secréteurs d'anticorps vers ceux qui seront capables de produire les anticorps monoclonaux répondant aux caractéristiques sus-indiquées.

Les anticorps monoclonaux anti-Leishmania selon l'invention sont caractérisés par leur capacité à inhiber l'infection de cellules sarcomateuses par les formes promastigotes de une ou plusieurs espèces de Leishmania, lorsque ces cellules sarcomateuses sont mises en contact avec lesdites formes promastigotes pré-incubées avec ces anticorps monoclonaux dans des conditions qui, en l'absence desdits anticorps monoclonaux, conduiraient à une parasitémie élevée dans lesdites cellules.

Un procédé d'obtention et d'isolement d'hybridomes secréteurs d'anticorps monoclonaux anti-leishmania possédant les propriétés sus-indiquées est caractérisé en ce que l'on met en contact les formes promastigotes infecteuses avec, d'une part, des anticorps monoclonaux reconnaissant des antigènes d'une ou plusieurs espèces de leishmanies et secrétés parmi des hybridomes préalablement formés de façon en soi connue entre des cellules de myélome et des cellules spléniques d'un animal, notamment la souris, préalablement immunisé contre une ou plusieurs souches de leishmanies et, d'autre part, avec des cellules sarcomateuses dans des conditions qui, en l'absence desdits anticorps, conduiraient à une infection parasitaire desdites cellules sarcomateuses, et en ce que l'on sélectionne ceux de ces anticorps monoclonaux qui, dans l'opération précédente, protègent complètement *in vivo* les cellules sarcomateuses vis-à-vis de l'infection par lesdites leishmanies, après leur injection dans le péritoine d'une souris sensible auxdites cellules sarcomateuses, plus particulièrement d'une souris Balb-C. De préférence—mais non nécessairement—les formes promastigotes mises en oeuvre sont prétraitées avec les anticorps monoclonaux dans la souris Balb-C. Ce prétraitement consiste de préférence en un contact maintenu à 37°C, pendant 30 minutes. L'infection éventuelle desdites cellules sarcomateuses peut être appréciée par prélèvement de l'ascite, lorsque celle-ci s'est formée dans la cavité péritonéale de la souris, mise en culture des cellules sarcomateuses de l'ascite dans un milieu approprié, par exemple le milieu NNN, et détection éventuelle des promastigotes formés dans le milieu. Il va sans dire que la sélection des hybridomes

# EP 0 197 062 B1

secréteurs est en rapport direct avec celle des anticorps monoclonaux protecteurs.

Il résulte de ce qui précède que l'invention met en jeu la capacité des anticorps monoclonaux finalement sélectionnés d'exercer une activité protectrice efficace vis-à-vis des cellules particulièrement sensibles à l'infection parasitaire. Les cellules sarcomateuses connues sous la désignation "TG180" décrites par Sartorelli A. C. et Both B. A. (1961) Federation Proceedings, *20*, 156—159, se sont révélées à ce point sensibles que l'on a proposé de les utiliser pour la production rapide et en grande quantité de leishmanies, plus particulièrement sous leur forme amastigote (forme intracellulaire des leishmanies). Les conditions de cette production et de l'isolement des leishmanies décrites ont été décrites par L. Monjour et Coll. dans l'article intitulé "Rapid, Large, Scale Production and Isolation for Leishmania Amastigotes", Annals of Tropical Medicine Parasitology (1184), *78*, 423—425. Par ailleurs ces cellules TG 180 produisent une ascite lorsqu'elles sont injectées à une souris Balb-C; par conséquent la moindre cellule sarcomateuse infectée amplifiera chez la souris la réponse à l'infection en multipliant les cellules infectées et en propageant l'infection au moins à toute l'ascite.

L'invention résulte donc d'une orientation de recherche basée sur l'hypothèse initiale qui a été faite (et qui s'est révélée féconde) qu'il devait être possible de produire et d'isoler des anticorps monoclonaux anti-Leishmania capables de protéger des cellules normalement aussi sensibles à l'infection parasitaire. Cette hypothèse allait donc également fournit l'essence du test particulièrement rigoureux qui constitue le fondement du procédé de production et de sélection des anticorps monoclonaux protecteurs conformes à l'invention.

L'invention concerne donc plus particulièrement les anticorps monoclonaux anti-leishmania ayant les caractéristiques essentielles de ceux qui sont sécrétés par des hybridomes obtenus par fusion de cellules de myélome SP2/0 et de cellules spléniques prélevées sur des souris Balb/c préalablement infectées avec des promastigotes de la souche de *L. infantum* déposée à la C.N.C.M. de 26 septembre 1984 sous le numéro I-342, les surnageants de culture des clones obtenus ou les liquides ascitiques correspondants étant triés pour ne retenir dans un premier stade (sur la majorité) que les anticorps monoclonaux qui fixent plusieurs leishmanies à la fois, avantageusement sur au moins l'une des parasites appartenant à chacune des espèces *L. infantum. L. tropica, L. brasiliensis* et *L. mexicana* (détection de la fixation par immunofluorescence indirecte globale) et, au terme d'un second tri, ceux des anticorps monoclonaux qui se fixent sur les parasites vivants ou tués, lesdits anticorps monoclonaux étant caractérisés:

en ce qu'ils reconnaissent des antigènes communs à plusieurs espèces de leishmanies, notamment *L. infantum, L. mexicana, L. tropica, L. donovani,* et *L. brasiliensis,*

par leur capacité à inhiber l'infection de cellules sarcomateuses de souris appartenant à la lignée TG180, lorsque $2.10^3$ des cellules de cette lignée sont injectées dans le péritoine de souris Balb/c, en mélange avec 60 microlitres d'ascite contenant les susdits anticorps monoclonaux et $10^5$ promastigotes d'une ou de plusieurs espèces de leishmanies qui avaient été incubés au préalable en présence desdits anticorps monoclonaux à 37°C pendant 30 minutes. En l'absence des anticorps monoclonaux protecteurs, un tel traitement conduirait à une parasitémie aiguë des cellules. Ces indications quantitatives déterminent également les conditions préférées du test de sélection qui est mis en oeuvre dans le procédé selon l'invention.

Les anticorps monoclonaux mis en oeuvre dans les opérations d'infection des cellules sarcomateuses en vue de la sélection des anticorps monoclonaux protecteurs résultent de préférence déjà d'une pré-sélection. Comme on l'a indiqué plus haut, les anticorps monoclonaux mis en oeuvre sont ceux qui reconnaissent des antigènes d'une ou plusieurs espèces de leishmania.

Cette pré-sélection peut être réalisée de toutes façons en soi connues, par exemple dans les conditions qui suivent (et dont d'ailleurs un exemple de mise en oeuvre détaillée sera indiqué plus loin dans l'exemple).

Partant d'une ou de plusieurs espèces choisies de parasites, on immunise un animal, notamment la souris, avec les parasites sous la forme promastigote et l'on réalise une fusion cellulaire des cellules spléniques de l'animal préalablement sacrifié avec des cellules de myélome appropriées, essentiellement selon la méthode décrite par Kohlere G. et Milstein C. (1975), Nature, *256*, 495—497. Les cellules hybrides productrices d'anticorps peuvent ensuite être clonées par toute méthode en soi connue, de préférence par la méthode des dilutions limites. Les hybridomes finalement sélectionnés sont ensuite mis en oeuvre dans la production d'ascites, de préférence par la technique décrite par Roseto A. et Coll. (1983), Journal of Virology, *64*: 237—244. La détermination des classes IgG est ensuite réalisée de préférence par la méthode d'immunodiffusion, décrite par Roseto A. et Coll. (1982), Comptes Rendus de l'Académie des Sciences Paris, *294*: 347—352. De préférence, on recueille les hybridomes secréteurs d'anticorps monoclonaux appartenant à la classe des immunoglobulines IgG et l'on trie les surnageants de culture des clones obtenus ou les liquides ascitiques correspondants, selon la méthode décrite par Monjour L. et Coll. dans "Annales de la Socitéé Belge de Médicine Tropicale" (1978), *58*: 293—300, pour ne retenir dans un premier stade (sur la majorité) que les anticorps monoclonaux qui fixent plusieurs leishmanies à la fois, avantageusement sur au moins l'un des parasites appartenant à chacune des espèces *L. infantum, L. tropica, L. brasiliensis* et *L. mexicana* (détection de la fixation par immunofluorescence indirecte globale) et, au terme d'un second tri, ceux des anticorps monoclonaux qui se fixent sur les parasites vivants ou tués.

Il va de soi que l'on peut également mettre en oeuvre tout autre protocole de production et de sélection d'anticorps monoclonaux aptes à reconnaître les parasites vivants ou tués. Il convient d'ailleurs de noter que la sélection des anticorps monoclonaux parmi ceux d'entre eux qui présentent les caractéristiques des immunoglobulines IgG n'est obligatoire en aucune façon.

La mise en oeuvre dans le procédé selon l'invention d'un ou de plusieurs anticorps monoclonaux anti-Leishmania ainsi préalablement sélectionnés peut conduire non seulement à des anticorps monoclonaux protecteurs contre celles des espèces de parasites initialement mises en oeuvre (à l'occasion de l'immunisation des animaux, dont les cellules spléniques ont ensuite été mises en oeuvre pour la production de ces anticorps monoclonaux), mais encore contre des espèces de leishmanies différentes. Il est à cet égard remarquable que la mise en oeuvre du procédé selon l'invention à partir de plusieurs espèces de parasites permet d'isoler des anticorps monoclonaux reconnaissant un ou plusieurs antigènes qui se sont avérées être communs à grand nombre d'espèces de leishmanies.

En particulier, la mise en oeuvre du procédé selon l'invention dans les conditions qui seront décrites plus loin permet d'isoler des anticorps monoclonaux reconnaissant des antigènes, protéines ou glycoprotéines ayant (pour certains de ces anticorps monoclonaux) des poids moléculaires de l'ordre de 40.000, 70.000 et 113.000 et, pour d'autres anticorps monoclonaux, des antigènes ou protéines ayant des poids moléculaires de l'ordre de 140.000 (précision de l'ordre de 10%), tous ces antigènes ou protéines pouvant être obtenus à partir de lysats de leishmanies, notamment dans les conditions qui seront rappelées plus loin.

Des essais semblables répétés sur d'autres lysats ont d'autre par fait apparaître que l'on pouvait aussi isoler à partir de lysats de ces diverses espèces de leishmanies des antigènes protecteurs (ou susceptibles d'induire la production *in vivo* d'anticorps protecteurs ayant des poids moléculaires beaucoup plus petits notamment inférieurs ou égaux à 30.000 kD, ou même inférieurs à 20.000 kD.

Les essais dont les résultats sont rapportés plus loin font apparaître la nature glucidique de certains des antigènes protecteurs concernant l'invention.

Ainsi a-t-il été constaté que l'antigène protecteur de 70 kD, qui peut être obtenu à partir des susdits lysats, contient des groupes glucose et mannose, et que des antigènes protecteurs présentant des poids moléculaires respectivement de 20 et 30—32 kD comportaient des groupes fucose et α-D-L-acétyl-galactosamine.

Le procédé selon l'invention peut donc, au niveau de l'infection des cellules sarcomateuses, être mis en oeuvre avec des anticorps monoclonaux dont a été reconnue la capacité, au cours de tris préalables, de reconnaître des antigènes d'espèces différentes. En alternative, on peut également mettre en oeuvre, au niveau de l'infection des mélanges d'anticorps monoclonaux obtenus à partir d'hybridomes présélectionnés et ayant mis en jeu des Leishmanies appartenant à des espèces différentes au niveau de l'immunisation initiale. Par exemple, on met en oeuvre au niveau de l'infection un mélange d'anticorps monoclonaux obtenus à partir d'hybridomes qui ont eux-mêmes été obtenus par hybridation entre des cellules de myélomes et des cellules spléniques provenant de souris qui avaient été immunisées respectivement par *L. mexicana, L. tropica, L. donovani, L. infantum* et *L. brasiliensis*. Lorsque l'on a déterminé la capacité d'un mélange déterminé d'anticorps monoclonaux de protéger les cellules sarcomateuses contre l'infection *in vivo* par une pluralité de parasites déterminés, on peut encore répéter l'essai de protection avec chacun des anticorps monoclonaux déterminés contenus dans le mélange, en vue de rechercher celui qui est plus particulièrement capable de protéger les cellules sarcomateuses contre l'ensemble desdits parasites et par conséquent d'isoler l'hybridome correspondant.

Les méthodes proposées ci-dessus ne sont qu'indicatives de toutes celles qui peuvent être mises en oeuvre pour mettre en évidence ou isoler les polypeptides portant les sites antigéniques intervenant dans la réaction antigène-anticorps. Les techniques de recombinaison génétique pourront également être mises en oeuvre aux mêmes fins.

Les polypeptides reconnus par des anticorps selon l'invention peuvent, surtout lorsqu'ils sont communs à plusieurs espèces de leishmanies, être utilisés pour la production de compositions pharmaceutiques destinées à la vaccination contre les leishmanioses. Ces polypeptides y sont alors associés avec les véhicules physiologiquement acceptables appropriés aux mèthodes de vaccination. Avantageusement ces compositions pharmaceutiques sont constituées de façon appropriée à l'administration par injection.

Des caractéristiques supplémentaires préférées de l'invention apparaîtront encore au cours de la description qui suit de conditions préférées qui ont été mises en oeuvre pour la production, la sélection et l'isolement d'hybridomes conformes à l'invention.

1—Parasites

On a utilisé les formes promastigotes des espèces suivantes: *L. infantum* LEM 497, *L. tropica* LEM 129, *L. brasiliensis guyanensis* LEM 311 et *L. mexicana amazonensis* LV 79 pour la caractérisation des antigènes reconnus par les anticorps. En plus de ces souches, les souches suivantes ont été utilisées pour les expériences de protection: *L. brasiliensis guyanensis*: Cay H 60 et Cay H 53; *L. donovani*: ANTWERPEN, ITMAP 430 (Portugal), ITMAP 263 (Maroc), ITMAP 240—366 (Tunisie), ITMAP 1356 (Italie), LV 9 (Ethiopie), LEM 139 (Inde), *L. mexicana mexicana* LEM 280, *L. tropica* LV 39.

2—Production de formes promastigotes infectieuses

$2 \times 10^3$ cellules sarcomateuses de souris (A. C. Sartorelli et B. A. Both., Ped. Proc., *20*, 1961, p.

156—159), dénommées TG 180 (P. Couzineau et H. Baufine-Ducroc, Ann. Par. Hum. Comp. *44*, 1969, 217—224) sont mélangées à $2\times10^6$ formes promastigotes. Après injection par voie intrapéritonéale à la souris Balb/c, un liquide d'ascite est recueilli 7 à 10 jours plus tard. Une centrifugation de ce liquide donne un culot de cellules sarcomateuses infectées par des formes amastigotes. Après 4 jours de culture en milieu NNN à 22°C, on obtient une prolifération de promastigotes constamment infectieux pour la souris Balb/c (L. Monjour, I. Vouldoukis, O. Brandicourt, D. Mazier, C. Alfred, I. Ploton et M. Gentilini, Ann. Trop. Med. Par. 1984, *78*, 423—425).

3—Production d'anticorps monoclonaux
   a) Immunisation des souris

Des souris Balb/c ont subi 3 injections souscutanées, espacées chacune de 15 jours, avec $10^6$ promastigotes de *L. infantum* mélangés à 10 microgrammes d'un immunoadjuvant, à base de saponine commercialisé sous la désignation QUIL A. Un moins plus tard, les souris ont subi une dernière injection intrapéritonéale de $2\times10^7$ promastigotes. 4 jours plus tard, les souris ont été sacrifiées et leurs cellules spléniques prélevées.

b) Hybridomes
   La fusion des susdites cellules spléniques et de cellules de myélome SP2/0, la culture des hybrides, la détection des anticorps monoclonaux, leur caractérisation par immunodiffusion radiale et la production d'ascites ont été effectuées selon des protocoles décrits précédemment (A. Roseto, T. F. Vautherot, P. Bobulesco et M. C. Guillemin, C. R. Acad. Sciences Paris—*294*, 1982, p. 347—352).

c. Mise en évidence des anticorps
   Les produits de secrétion des hybridomes ont été éprouvés sur des promastigotes de l'Ancien et du Nouveau monde, morts ou vivants, et ce, par immunofluorescence indirecte (L. Monjour, C. Mille, P. Druilhe et M. Gentilini, Ann. Soc. Belge Med. Trop., *58*, 1978, p 293—300). 10 ascites ont produit une réaction positive contre au moins certaines des espèces de parasites identifiés dans le tableau plus loin. 5 ascites se sont révélées produire une réaction positive contre chacune desdites espèces de parasites.

4—Epreuves d'inhibition
   Ces épreuves ont été menées sur plusieurs espèces de leishmanies de l'Ancien et du Nouveau monde. 5 anticorps monoclonaux de l'isotope IgG1, produits en ascite et réagissant positivement en immunofluorescence sur les promastigotes, ont été sélectionnés, puis mélangés pour les essais d'inhibition. 60 microlitres du mélange ont été mis en contact avec $10^5$ promastigotes d'une espèce, à 37°C, pendant 30 minutes. $2\times10^3$ cellules TG 180 ont alors été ajoutées; après un lavage, le mélange a été injecté intrapéritonéalement à la souris Balb/c. 10 jours plus tard, l'ascite formée est prélevée par aspiration à la seringue. La recherche des amastigotes intra et extracellulaires se fait sur une préparation colorée au May-Grènwald-Giemsa. La présence de promastigotes est rélévée après 20 jours d'incubation des cellules ascitiques en milieu NNN. Les contrôles expérimentaux sont les suivants: anticorps monoclonaux anti-*Plasmodium falciparum* et anti-HBs, surnageants d'ascites provoqués par l'injection de pristane et de myélome SP2/0; un autre contrôle consiste à mettre en contact l'ascite induite par le sarcome TG 180 avec chaque espèce de la panoplie des leishmanies, à 37°C, pendant 30 minutes. Enfin, le caractère pathogène des espèces est prouvé par l'infection, dans les mêmes conditions, de cellules sarcomateuses avec chaque espèce de Leishmanie.

5—Caracterisation antigenique
   Des lysats des différents parasites, réalisés en présence d'inhibiteurs de protéases (PMSF et TLCK à $10^{-3}$M) ont été analysés, par immunoempreinte sur feuille de nitrocellulose (H. Towbin, T. Staehelin et I. Gordon, PNAS, USA, *76*, 1979, p. 4350—4354) du profil électrophorétique obtenu par la technique de Laemmli (V. K. Laemmli, Nature, *227*, 1970, p. 680—683) en gel d'acrylamide à 10%. Les complexes antigèes-anticorps ont été révélés par des anti-anticorps marqués à la péroxydase selon Avrameas (S. Avrameas et B. Guilbert, Eur, J. Imm., *1*, 1971, 394—396).
   Les poids moléculaires des antigènes ont été appréciés d'après leurs distances de migration dans le gel, comparées à celles des protéines suivantes de poids moléculaires connus: ferritine (220 kD), ovotransférine (77 kD), albumine bovine (67 kD), catalase (60 kD), ovalbumine (45 kD), lactate déshydrogénase (36 kD), chymotrypsinogène A (25,7 kD), ferritine (forme monomère) (18,5 kD), myoglobuline (17,2 kD), cytochrome c (12,3 kD).
   Dans d'autres essais, on a utilisé les protéines de référence suivantes: thyroglobuline (330 kD, ferritine (220 kD), phosphorylase B (94 kD), albumine bovine (67 kD), catalase (60 kD), ovalbumine (43 kD), lactate déshydrogénase (36 kD), anhydrase carbonique (30 kD), un "inhibiteur" (20,1 kD), ferritine (forme monomère) (18,5 KD), lactalbumine (14,4 kD). Toutes ces protéines sont commercialisées par Pharmacia.

6—Serums humains
   Les sérums de deux malades atteints de leishmaniose viscérale, confirmée par la présence de parasites à l'examen direct, ont permis de compléter selon le protocole précité, la carte antigénique. Il s'agissait de deux infections, contractées en zone méditérranéenne (Hérault et Algérie), ayant évolué respectivement pendant 3 et 6 mois.

Resultats:
1—Choix des anticorps monoclonaux
Après clonage par dilution limite des hybrides positifs, seuls 5 hybridomes secrétant des anticorps d'isotype IgG1 ont été retenus. En immunofluorescence, ils marquent la membrane des promastigotes vivants. Ces anticorps ont ensuite été produits dans des ascites de souris.

2—Epreuves d'inhibition
L'effect inhibiteur des anticorps monoclonaux sur le développement des 4 espèces de Leshmanies a été révélé par l'absence d'amastigotes dans les cellules sarcomateuses injectées par voie intrapéritonéale. Dix jours après l'injection, le transfert des cellules en milieu NNN révèle pas la présence de formes promastigotes. En revanche, les couplages réalisés avec les ascites négatives ou des anticorps monoclonaux non spécifiques se traduisent par une infection constante, prouvée secondairement par la multiplication des promastigotes in vitro.

3—Caracterisation des antigens
Les antigènes des différentes espèces de Leishmanies pouvant être reconnus par le mélange des anticorps monoclonaux inhibiteurs, ont été répertoriés dans le tableau ci-après:

TABLEAU

| Espèces | L. infantum | L. tropica | L. mexicana amazonensis | L. brasiliensis guyanensis |
|---|---|---|---|---|
| poids | 113 | 113 | 113 | 113 80 |
| moléculaire | 70 68 48 | 70 | 70 60 44 | 70 60 44 |
| (en kilo-daltons) | 44 40 | 40 | 40 36 | 40 36 |

Les nombres soulignés indiquent une forte réaction entre l'antigène et l'anticorps. Il est important de souligner que trois antigènes de poids moléculaire 40 kD, 70 kD et 113 kD sont constamment reconnus dans la panoplie des leishmannies de l'Ancien et du Nouveau monde. Les anticorps leur correspondant permettant de prévenir l'infection par les promastigotes chez la souris, on peut penser que l'un ou l'autre de ces antigènes peut jouer un rôle dans l'acquisition d'une immunoprotection croisée. A titre comparatif, les résultats des immuno-empreintes réalisées avec les sérums des deux malades leishmaniens ont été également colligés. Ils mettent également en évidence un antigène de 70 kD, mais non ceux de 40 kD et 113 kD. La reconnaissance de plus de 5 antigènes chez L. mexicana amazonensis et L. brasiliensis guyanensis suggère des parentés antigéniques entre les différents constituants de la souche.
Il apparaît donc que l'antigène de 70 kD est:
1°) immunogène tant chez la souris que chez l'homme,
2°) présent dans toutes les souches de leishmanies étudiées.
De même ont été isolés des anticorps monoclonaux qui se sont avérés reconnaître une molécule de poids moléculaire d'environ 140.000 dans des lysats de plusieurs leishmanies. Comme le montrent encore les résultats d'essais supplémentaires, on peut également isoler par le procédé selon l'invention des hybridomes secréteurs d'anticorps monoclonaux reconnaissant des antigènes ayant des poids moléculaires inférieurs à 20.000 et aptes à leur tour d'induire la production in vivo d'anticorps protecteurs contre les Leishmania.

4—Essais de vaccination
Expériences de vaccination chez la souris Balb/c avec des extraits de promastigotes de L. infantum ont été menés de la façon suivante:
Les prosmastigotes ont été lysés ainsi: le culot de parasites a été suspendu dans du tampon Tris 10 mM pH 7.5 contenant des détergents du type NP40 (0,5%) et SDS (1%). La suspension obtenue a ensuite été traitée par ultrasons (soniquée) pendant 1 minute 30 secondes.

L'homogénat a été repris dans du "tampon d'échantillonnage" ("sample buffer") contenant:

5% de β-mercapto-éthanol,

3% de SDS,

0,065 M Tris et

10% de glycérol,

et déposé sur un gel de polyacrylamide et de dodécylsulfate de sodium à 10% d'acrylamide (PAGE-SDS). Après électrophorèse, une partie du gel a été colorée afin de révéler les bandes protéiques et une autre partie, non colorée, a été découpée en 6 fractions nommées F1, F2...F6.

Les fourchettes ou plages de poids moléculaires correspondant à chaque fraction ont été les suivantes (poids moléculaires appréciés d'après les distances de migration desdites protéines, comparées à celles des protéines de poids moléculaires utilisées dans les expériences décrites dans le paragraphe 5 ci-dessus.

| Fractions | Plages de poids moléculaire |
|-----------|------------------------------|
| F1 | >94.000 daltons |
| F2 | entre 94.000 et 67.000 daltons |
| F3 | entre 67.000 et 43.000 daltons |
| F4 | entre 43.000 et 30.000 daltons |
| F5 | entre 30.000 et 20.000 daltons |
| F6 | en dessous de 20.000 daltons. |

Chaque fraction a été électroéluée du gel, puis dialysée, et enfin:

soit lyophilisée,

soit conservée telle quelle à −80°C.

L'activité protectrice éventuelle de chacune de ces fractions a été évaluée par la capacité de ces fractions à induire *in vivo* la production d'anticorps aptes à inhiber *in vivo* l'infection parasitaire de cellules sarcomateuses TG180, dans des essais réalisés selon le protocole décrit dans le paragraphe 4 ci-dessus si ce n'est que des parasites ont été préincubés avec des sérums obtenus à partir des animaux (souris) immunisés avec les susdites fractions, avant d'être mis en contact avec les cellules qui ont ensuite été injectées à des souris Balb/c.

On indique ci-après les conditions dans lesquelles les deux lots de souris utilisées ont été immunisées.

a) Par voie sous-cutanée (5 souris/fraction).

Chaque souris a reçu 3 injections à un mois d'intervalle, de 100 mg de fraction du stock maintenu à −80°C avec 100 µg de muramyldipeptide (MDP).

Les contrôles ont été faits avec du NaCl 0,15 M+MDP 100 µg.

b) Par voie intraveineuse (8 souris/fraction)

Chaque souris a reçu 3 injections à un mois d'intervalle de 100 µg de fraction du stock lyophilisé. Les contrôles ont été faits avec NaCl 0,15 M.

Dans tous les cas, 3 mois après l'immunisation, les sérums des animaux immunisés ont été prélevés et le sérum de chaque animal a été décomplémenté, puis utilisé dans le test de protection précédemment décrit dans le brevet principal.

On a ainsi testé l'effect protecteur de chaque sérum de souris sur un mélange de 4 souches de *Leishmania (L. infantum, L. mexicana amazonensis, L. tropica* et *L. brasiliensis guyanensis).*

Dans le tableau qui suit et dans lequel sont indiqués les effets protecteurs induits par les sérums obtenus chez les animaux préalablement immunisés par les fractions F1 à F6, on entend par "protection complète avec tous les sérums", la capacité pour les sérums en cause d'inhiber l'infection parasitaire des cellules sarcomateuses de la lignée TG180 dans tous les animaux des groupes concernés, lorsque $2 \times 10^3$ cellules de cette lignée sont injectées dans le péritoine des souris immunisées en mélange avec $10^5$ promastigotes non prétraités par les sérums.

Les résultats sont résumés dans le tableau ci-après:

| Fraction | Effet protecteur |
|----------|------------------|
| F1 | pas de protection constante |
| F2 | protection complète avec tous les sérums |
| F3 | pas de protection constante |
| F4 | pas de protection constante |
| F5 | protection complète avec tous les sérums |
| F6 | protection complète avec tous les sérums |
| contrôle | pas de protection du tout. |

Ces résultats sont identiques tant avec les sérums de souris immunisées par voie intraveineuse que par voie sous-cutanée.

Par ailleurs on a caractérisé la nature glucidique des différentes fractions obtenues à partir de lysat de *L. infantum.*

Pour cela, un lysat de *L. infantum* a été résolu sur gel de PAGE-SDS à 10% d'acrylamide, dans les mêmes conditions que précédemment, puis le profil électrophorétique a été transféré sur feuille de nitrocellulose.

La feuille de nitrocellulose a été découpée en plusieurs bandes de papier comportant toutes, après transfert, le susdit profil électrophorétique. Chaque bande de papier a fait l'objet d'une révélation à l'aide de lectines conjuguées à de la péroxydase et fabriquées par la technique de J. L. Guesdon et Col. (J. of Immmunological Methods *39* (1980), 1—13). Les conjugés suivants ont été fabriqués:

1) Concanavaline lectine conjuguée—péroxydase,
2) Ulex europaeus-péroxydase,
3) Dolichos biflorus—péroxydase, ces différentes lectines étant caractérisées par des affinités marquées respectivement pour:

1) le mannose, glucose,
2) le fucose et
3) l'α-D-N-acétyl-galactosamine.

Le papier est incubé 30 minutes avec une dilution au 1/200e de la lectine conjuguée, puis après plusieurs lavages en présence de l'agent mouillant Tween 20 à 0,1%, on a révélé la présence de lectine fixée par la présence de la péroxydase associée, en ajoutant les substrates de cette dernière (eau oxygénée et diaminobenzidine). La présence d'un précipité marron sur le papier permet la localisation de l'activité enzymatique.

Les résultats sont les suivants:

| lectine | bande reconnue | sucre concerné |
|---|---|---|
| ConA-PO | 85 kDaltons* | glucose, mannose |
| Ulex-PO | 85 kD | fucose |
| | 30 kD* | |
| | 20 kD* | |
| Dolichos-PO | 85 kD | α-D-N-acétyl-galactosamine |
| | 67 kD | |
| | 32 kD* | |
| | 20 kD* | |

Les poids moléculaires sont estimés à 10%.

Les résultats marqués d'une astérique concernent des bandes qui se sont révélées être reconnues par les anticorps des animaux immunisés avec les fractions contenant les poids moléculaires correspondants.

L'invention concerne par conséquent également les compositions immunogènes caractérisées par l'association de l'une au moins des fractions actives sus-indiquées avec un excipient physiologiquement acceptable permettant son administration à un hôte vivant, en vue de lui conférer une immunité à l'égard non seulement de ces fractions polypeptidiques, mais également des parasites pathogènes.

On remarquera d'ailleurs que des sérums isolés à partir de 20 malades ont montré que les sérums de 2 malades seulement reconaissaient la glycoprotéine de 70 kD. Ce fait tend à montrer que, pour être protégés contre les leishmanioses, les organismes humains doivent normalement contenir des anticorps formés contre la glycoprotéine de 70 kD. En effet, l'absence d'anticorps correspondant dans les sérums de malades s'explique par la lenteur de la formation des anticorps protecteurs.

A l'inverse, les différentes fractions selon l'invention peuvent également être utilisées dans un procédé de détection de la présence d'anticorps antileishmanies, notamment dans des échantillons sanguins provenant de l'homme ou de l'animal, par exemple pour vérifier l'existence ou non d'une protecton de l'hôte contre les leishmanioses.

Les anticorps monoclonaux selon l'invention peuvent être également utilisés en tant que véhicules pour transporter une molécule toxique (par exemple la gélonine) ou un médicament anti-parasitaire et le concentrer sur les sites mêmes de son action, plus particulièrement les parasites eux-mémes sous leur forme promastigote ou amastigote. Ansi, les anticorps monoclonaux selon l'invention pouvent-ils être couplés chimiquement de façon covalente avec des substances toxiques ou des substances médicamenteuses anti-leishmania. Ce couplage covalent mettra naturellement en jeu des groupes fonctionnels tant de l'anticorps que des médicaments utilisés qui n'interviennent pas dans la réaction antigène-anticorps, d'une part, au niveau de l'action des substances, d'autre part. Des médicaments appropriés ont été envisages, dans la demande internationale WO83/01785. Dans une forme avantageuse de conjugés susceptibles d'être utilisés, le médicament est encapsulé dans des liposomes, ceux-ci étant eux-mêmes liés de façon covalente à l'anticorps, par exemple par l'intermédiaire du N-succinimidyl-3-(2-pyridyl-dithio)-propionate (SPDP): voir notamment J. Biol. Chem. *255*, 2015—2018 (1980); Nature, vol. 293, no. 5829, p. 228, 1981 et J. of Supramolecular Structure and Cellular Biochemistry 16, p. 243—258 (1981).

Les anticorps monoclonaux selon l'invention peuvent également être utilisés pour purifier des compositions polypeptidiques susceptibles de contenir des protéines immunogènes, telles que les antigènes de poids moléculaires 40.000, 70.000, 113.000 ou 140.000 daltons, selon la nature de l'anticorps utilisé. Ce procédé est avantageusement appliqué à des extraits obtenus à partir des parasites (par exemple lysats de parasites). Il peut aussi être appliqué à l'extraction de ces polypeptides des surnageants de milieux de culture de ces parasites, dès lors que ces surnageants sont susceptibles de contenir de tels polypeptides en tant que produits du métabolisme desdits parasites. Pour la mise en oeuvre de ce procédé, les anticorps monoclonaux sont avantageusement immobilisés sur un support solide, de préférence adapté à des opérations de chromatographie d'affinité. Par exemple, ces anticorps monoclonaux sont fixés sur un réseau d'agarose à réticulation tri-dimensionnelle, commercialisé sous la marque Sepharose par la société suédoise Pharmacia A. G., par exemple par la méthode au bromure de cyanogène.

L'invention concerne donc plus particulièrement un procédé de séparation de ces antigènes caractérisés par les opérations consistant à faire passer le milieu susceptible de les contenir (notamment extrait de parasite ou milieu de croissance de ces parasites) au contact d'une colonne d'affinité portant les susdits anticorps monoclonaux, pour fixer sélectivement lesdits polypeptides, puis à récupérer ceux-ci par dissociation du complexe antigène-anticorps au moyen d'un tampon approprié, notamment d'une solution de force ionique adéquate, notamment d'un sel, de préférence l'acétate d'ammonium (lequel le laisse pas de résidu lorsque l'on réalise ensuite la lyophilisation de la préparation). On peut également avoir recours à une solution acidifiée à pH 3—4 ou à un tampon glycine au même pH.

L'invention concerne également les polypeptides communs à plusieurs Leishmanies, et plus particulièrement ceux qui sont reconnus par les anticorps monoclonaux secrétés par les souches d'hybridomes qui ont été déposées à la Collection Nationale des Cultures de Micro-organismes (C.N.C.M.) de l'Institut Pasteur de Paris, sous les no. I-344 et I-345 le 1er octobre 1984.

L'invention concerne plus particulièrement encore parmi ces polypeptides ceux d'entre eux, notamment celui qui présente un poids moléculaire de l'ordre de 70.000—ou ceux ayant des fourchettes de poids moléculaires 67.000—94.000 daltons, ou encore ceux ayant des poids moléculaires inférieurs à 20.000 daltons, qui sont également reconnus par des sérums provenant de malades humains affectés par des leishmanioses.

Ces polypeptides ou antigènes sont eux-mêmes susceptibles d'être utilisés comme réactifs, ou même comme agents de diagnostic *in vitro*, pour la détection d'anticorps anti-Leishmania. Il va de soi que l'invention concerne également des fractions polypeptidiques pouvant avoir des poids moléculaires plus faibles, dès lors qu'ils porteraient des sites antigéniques susceptibles d'être reconnus par les mêmes anticorps monoclonaux. Il apparaîtra clairement au spécialiste qu'à partir de l'instant où l'on dispose des anticorps monoclonaux selon l'invention, on peut envisager l'isolement, à partir des antigènes sus-indiqués, des séquences peptidiques plus petites contenant les mêmes sites antigéniques, par exemple en ayant recours à des techniques en soi connues de découpage du polypeptide initiale par des enzymes susceptibles de découper les polypeptides plus grands en des sites spécifiques. A titre d'exemple de telles protéines, on peut mentionner l'enzyme de *Staphylococcus aureus* V8, l'alpha-chymotrypsine, la "protéase de glande sous-maxillaires de souris" (mouse submaxillary gland protease) commercialisée par la société Boehringer, la collagénase de *Vibrio alginolyticus chemovar iophagus*, qui reconnait spécifiquement lesdits peptides Gly-Pro et Gly-Ala, etc.

Les polypeptides du genre en question peuvent également être utilisés pour séparer des anticorps présentant les caractéristiques sus-indiquées à partir d'un mélange polyclonal d'anticorps. Dans ce cas, les polypeptides du genre en question seront à leur tour immobilisés sur un support de chromatographie d'affinité, par exemple du genre indiqué plus haut. Le procédé de séparation comprendra par conséquent l'étape consistant à faire passer une solution contenant les anticorps polyclonaux au contact des polypeptides immobilisés, et l'étape de récupération des anticorps retenus au moyen d'une solution ou d'un tampon analogue à celle ou celui qui a été envisagé plus haut.
des antigènes immunogènes sus-indiqués avec un excipient physiologiquement acceptable permettant son administration à un hôte vivant, en vue de lui conférer une immunité à l'égard desdits polypeptides. Ces polypeptides constituent des principes actifs dont l'immunogénicité peut être mise en oeuvre à chaque fois qu'est recherchée une protection *in vivo* contre des parasites.

L'invention concerne également un procédé utilisant les antigènes reconnus par les susdits anticorps monoclonaux pour la détection de la présence d'anticorps anti-Leishmania, notamment dans des échantillons sanguins provenant de l'homme ou de l'animal, en vue d'effectuer la détection de l'affection.

Dans une variante de ce procédé, la détection de la présence d'anticorps anti-Leishmania met en jeu des réactions d'hypersensibilité (réactions intradermiques) nécessaires au diagnostic de l'affection chez l'homme ou l'animal.

Comme il va de soi et comme il résulte d'ailleurs déjà de ce qui précède, l'invention ne se limite nullement à ceux de ses modes d'application et de réalisation qui ont été plus spécialement envisagés; elle en embrasse au contraire toutes les variantes; en alternative, l'invention concerne un procédé utilisant l'infection des cellules sarcomateuses TG180 par les parasites (forme amastigote) pour déterminer l'efficacité de nouvelles formes d'anticorps (mono- ou polyclonaux) pour juguler l'infection parasitaire. Le

# EP 0 197 062 B1

même test peut être utilisé pour déterminer l'efficacité de nouvelles molécules médicamenteuses anti-Leishmaniose.

La souche de cellule sarcomateuse TG180 a été déposée à la CNCM (Collection Nationale des Cultures de Micro-organismes de l'Institut Pasteur) sous le numéro I-343, le 26 septembre 1984.

La souche de parasite *L. infantum* (LEM 497) qui a été utilisée dans l'immunisation initiale des souris en vue de la production des anticorps monoclonaux plus particulièrement décrits dans les exemples, a été déposée à la CNCM le 26 septembre 1984 sous le numéro I-342.

Il est entendu que lorsque l'on a fait usage du mot "leishmanies", c'est naturellement des parasites du genre Leishmania qu'il s'agissait.

## Revendications

1. Anticorps monoclonaux anti-leishmania ayant les caractéristiques essentielles de ceux qui sont sécrétés par des hybridomes obtenus par fusion de cellules de myélome SP2/0 et de cellules spléniques prélevées sur des souris Balb/c préalablement infectées avec des promastigotes de la souche de *L. infantum* déposée à la C.N.C.M. le 26 septembre 1984 sous le numéro I-342, les surnageants de culture des clones obtenus ou les liquides ascitiques correspondants étant triés pour ne retenir dans un premier stade (sur la majorité) que les anticorps monoclonaux qui fixent plusieurs leishmanies à la fois, avantageusement sur au moins l'un des parasites appartenant à chacune des espèces *L. infantum*, *L. tropica*, *L. brasiliensis* et *L. mexicana* (détection de la fixation par immunofluorescence indirecte globale) et, au terme d'un second tri, ceux des anticorps monoclonaux qui se fixent sur les parasites vivants ou tués, lesdits anticorps monoclonaux étant caractérisés:

en ce qu'ils reconnaissent des antigènes communs à plusieurs espèces de leishmanies, notamment *L. infantum*, *L. mexicana*, *L. tropica*, *L. donovani*, et *L. brasiliensis*,

par leur capacité à inhiber l'infection de cellules sarcomateuses de souris appartenant à la lignée TG180, lorsque $2.10^3$ des cellules de cette lignée sont injectées dans le péritoine de souris Balb/c, en mélange avec 60 microlitres d'ascite contenant les sudits anticorps monoclonaux et $10^5$ promastigotes d'une ou de plusieurs espèces de leishmanies qui avaient été incubés au préalable en présence desdits anticorps monoclonaux à 37°C pendant 30 minutes.

2. Anticorps monoclonaux selon la revendication 1, caractérisés en ce qu'ils sont sécrétés par des hybridomes qui ont été déposés à la C.N.C.M. respectivement sous les no. I-344 et I-345 le 1er octobre 1984.

3. Anticorps monoclonaux selon la revendication 1 ou 2, caractérisés en ce qu'ils reconnaissent des protéines ayant des poids moléculaires:

de moins 20.000, ou

de l'ordre de 30.000,

ou de l'ordre de 40.000,

de 67.000 à 94.000, notamment de l'ordre de 70.000,

de l'ordre de 113.000,

contenues dans des lysats de Leishmanies.

4. Anticorps monoclonaux selon la revendication 1 ou 2, caractérisés en ce qu'il reconnaissent des protéines ayant des poids moléculaires de l'ordre de 140.000 et contenues dans des lysats de Leishmanies.

5. Anticorps monoclonaux selon l'une quelconque des revendications 1 à 4, caractérisés en ce qu'il reconnaissent des protéines qui sont également reconnues *in vitro* par des sérums provenant de malades atteints de leishmaniose.

6. Hybridomes sécréteurs, caractérisés par le fait que les anticorps monoclonaux qu'ils sécrétent sont conformes à ceux définis dans l'une quelconque des revendications 1 à 5.

7. Procédé d'obtention et d'isolement d'hybridomes sécréteurs selon la revendication 6, caractérisé en ce que l'on met en contact les formes promastigotes infectieuses d'une espèce de Leishmanie avec, d'une part, des anticorps monoclonaux sécrétés par des hybridomes obtenus par fusion de cellules de myélome SP2/0 et de cellules spléniques prélevées sur des souris Balb/c préalablement infectées avec des promastigotes de la souche de *L. infantum* déposée à la C.N.C.M. le 26 septembre 1984 sous le numéro I-342, et, d'autre part, avec des cellules sarcomateuses dans des conditions qui, en l'absence desdits anticorps, conduiraient à une infection parasitaire desdites cellules sarcomateuses, et en ce que l'on sélectionne ceux de ces anticorps monoclonaux qui, dans l'opération précédente, protègent complètement *in vivo* les cellules sarcomateuses vis-à-vis de l'infection par lesdites Leishmanies, après leur injection dans le péritoine d'une souris sensible auxdites cellules sarcomateuses plus particulièrement d'une souris Balb/c.

8. Procédé selon la revendication 7, caractérisé en ce que les cellules sarcomateuses utilisées appartiennent à la lignée des cellules sarcomateuses de souris dénommées TG180.

9. Antigènes de parasites des leishmanioses ayant des poids moléculaires de l'ordre soit d'au plus 20.000, soit de l'ordre de 40.000, ou 70.000, ou 113.000, ou 140.000 daltons, ces polypeptides étant caractérisés:

en ce qu'ils sont reconnus par les anticorps monoclonaux tels que définis dans la revendication 1, notamment des anticorps monoclonaux secrétés par les hybridomes déposés à la C.N.C.M. sous les no. I-344 et I-345 le 1er octobre 1984,

11

par leur capacité à induire *in vivo* la production d'anticorps aptes à inhiber l'infection de cellules sarcomateuses de souris appartenant à la lignée TG180, lorsque 2.10³ cellules de cette lignée sont injectées dans le péritoine de souris Balb/c, en mélange avec 10⁵ promastigotes d'une ou de plusieurs espèces de Leishmanies qui, le cas échéant, ont été incubés au préalable avec les susdits anticorps.

10. Antigènes selon la revendication 9, caractérisés en ce qu'ils sont reconnus par des sérums humains contenant des anticorps à l'égard des Leishmanies.

11. Antigènes selon la revendication 9, ou la revendication 10, caractérisés en ce qu'ils contiennent un glycoprotéine protectrice ayant un poids moléculaire de l'ordre de 70 kD et comportent des groupes glucose et mannose.

12. Antigènes selon la revendication 9 ou la revendication 10, caractérisés en ce qu'ils contiennent des glycoprotéines isolables à partir de lysats de Leishmanies présentant des poids moléculaires de l'ordre de 20 kD et contenant des groupes fucose et α-D-N-acétyl-galactosamine.

13. Antigènes selon la revendication 9 ou la revendication 10, caractérisés en ce qu'il contiennent des glycoprotéines isolables à partir de lysats de Leishmanies présentant des poids moléculaires de l'ordre de 30—32 kD et contenant des groupes fucose et α-D-N-acétyl-galactosamine.

14. Composition pour la production de vaccins, pour l'homme ou l'animal, caractérisée par l'association d'un ou plusieurs polypeptides selon l'une quelconque des revendications 9 à 13, avec un véhicule pharmaceutiquement approprié à la vaccination, de préférence par voie parentérale.

15. Procédé pour la détection ou le diagnostic *in vitro* de l'infection par les Leishmanies chez l'homme ou l'animal, caractérisé en ce qu'il comprend la mise en contact de cellules, provenant notamment d'une biospie tissulaire (moelle osseuse, rate ou foie), avec des anticorps monoclonaux tels que définis dans l'une quelconque des revendications 1 à 5, et la détection, notamment par immunofluorescence, d'une réaction entre lesdits anticorps monoclonaux et lesdites cellules en cas d'infection.

16. Procédé utilisant les antigènes selon l'une quelconque des revendications 9 à 13, permettant de détecter *in vitro* la présence d'anticorps antileishmania dans le sérum d'un patient humain ou animal et d'effecteur ainsi le diagnostic *in vitro* de l'infection chez l'homme ou l'animal.

**Patentansprüche**

1. Monoklonale Anti-Leishmania-Antikörper mit den wesentlichen Merkmalen derer, die von Hybridomen ausgeschieden werden, die durch Fusion von Myelomzellen SP2/0 und Milzzellen erhalten wurden, entnommen von Balb/c-Mäusen, die zuvor mit Promastigoten des Stammes *L. infantum*, hinterlegt bei der C.N.C.M. am 26. September 1984 unter der Nummer I-342, infiziert wurden, wobei die Überstände der Kultur der erhaltenen Klone oder die entsprechenden aszitischen Flüssigkeiten aufgetrennt werden, so daß in einer ersten Stufe (in der Mehrheit) nur die monoklonalen Antikörper zurückbehalten werden, die mehrere Leishmanien auf einmal fixieren, vorteilhafterweise auf mindestens einem der Parasiten, die zu einer der Spezies *L. infantum, L. tropica, L. brasiliensis* und *L. mexicana* gehören (Nachweis der Fixierung durch globale indirekte Immunofluoreszenz) und, nach einer zweiten Auftrennung, diejenigen der monoklonalen Antikörper, die sich auf die lebenden oder getöteten Parasiten fixieren, wobei die genannten monoklonalen Antikörper charakterisiert sind:

dadurch, daß sie die mehreren Speizies von Leishmanien, insbesondere *L. infantum, L. mexicana, L. tropica, L. donovani* und *L. brasiliensis* gemeinen Antigene erkennen,

durch ihre Fähigkeit zum Inhibieren der Infektion von sarkomatösen Zellen von Mäusen, die zur Linie TG180 gehören, wenn 2.10³ Zellen dieser Linie ins Bauchfell von Balb/c-Mäusen injiziert werden, in Mischung mit 60 Mikrolitern von aszitischer Flüssigkeit, die die genannten monoklonalen Antikörper enthält und 10⁵ Promastigoten von einer oder mehreren Spezies von Leishmanien, die zuvor in Gegenwart der genannten monoklonalen Antikörper bei 37°C für 30 Minuten inkubiert wurden.

2. Monoklonale Antikörper gemäß Anspruch 1, dadurch gekennzeichnet, daß sie von Hybridomen ausgeschieden werden, die bei der C.N.C.M. unter den entsprechenden Nr. I-344 und I-345 am 1. Oktober 1984 hinterlegt wurden.

3. Monoklonale Antikörper gemäß Anspruch 1 oder 2, dadurch gekennzeichnet, daß sie Proteine erkennen, die Molekulargewichte aufweisen:

mindestens von 20 000 oder
der Größenordnung von 30 000,
oder der Größenordnung von 40 000,
von 67 000 bis 94 000, insbesondere der Größenordnung von 70 000,
der Größenordnung von 113 000,

die in den Lysaten von Leishmanien enthalten sind.

4. Monoklonale Antikörper gemäß Anspruch 1 oder 2, dadurch gekennzeichnet, daß sie Proteine erkennen, die Molekulargewichte in der Größenordnung von 140 000 aufweisen und in den Lysaten von Leishmanien enthalten sind.

5. Monoklonale Antikörper gemäß einem der Ansprüche 1 bis 4, dadurch gekennzeichnet, daß sie Proteine erkennen, die gleichermaßen *in vitro* durch Seren erkannt werden, die von mit Leishmaniose befallenen Kranken stammen.

6. Sekretorische Hybridome, gekennzeichnet durch die Tatsache, daß die monoklonalen Antikörper die sie ausscheiden, mit denen übereinstimmen, die in einem der Ansprüche 1 bis 5 definiert sind.

7. Verfahren zur Herstellung und Isolierung von sekretorischen Hybridomen gemäß Anspruch 6, dadurch gekennzeichnet, daß promastigote infektiöse Formen einer Spezies von Leishmanien in Kontakt gebracht werden mit, einerseits, monoklonalen Antikörpern, ausgeschieden von Hybridomen, die durch Fusion von Myelomzellen SP2/0 und Milzzellen erhalten wurden, entnommen von Balb/c-Mäusen, die zuvor mit Promastigoten des Stammes *L. infantum*, hinterlegt bei der C.N.C.M. am 26, September 1984 unter der Nummer I-342, infiziert wurden und andererseits, mit sarkomatösen Zellen unter Bedingungen, die in Abwesenheit der genannten Antikörper, zu einer parasitären Infektion der genannten sarkomatösen Zellen führen würden, und indem diejenigen der monoklonalen Antikörper ausgewählt werden, die, beim vorhergehenden Arbeitsgang, *in vivo* die sarkomatösen Zellen vollständig gegen die Infektion durch die genannten Leishmanien schützen, nachdem sie in das Bauchfell einer Maus injiziert wurden, die auf die genannten sarkomatösen Zellen empfindlich ist, insbesondere einer Balb/c-Maus.

8. Verfahren gemäß Anspruch 7, dadurch gekennzeichnet, daß die verwendeten sarkomatösen Zellen zur Linie der sarkomatösen Zellen der mit TG180 benannten Mäuse gehören.

9. Antigene von Parasiten der Leishmaniosen, die Molekulargewichte der Größenordnung von mehr als 20 000 aufweisen, entweder der Größenordnung von 40 000 oder 70 000 oder 113 000 oder 140 000 Dalton, diese Polypeptide sind charakterisiert:

dadurch, daß sie von den monoklonalen Antikörpern, wie sie im Anspruch 1 definiert sind, erkannt werden, insbesondere von monoklonalen Antikörpern, ausgeschieden von den Hybridomen, die bei der C.N.C.M. unter den Nr. I-344 und I-345 am 1. Oktober 1984 hinterlegt wurden,

durch ihre Fähigkeit, *in vivo* die Produktion von Antikörper auszulösen, die geeignet sind, die Infektion von sarkomatösen Zellen, von Mäusen, die zur Linie TG180 gehören, zu inhibieren, wenn $2.10^3$ Zellen dieser Linie in das Bauchfell von Balb/c-Mäusen injiziert werden, in Mischung mit $10^5$ Promastigoten von einer oder mehreren Spezies von Leishmanien, die, gegebenenfalls, zuvor mit den genannten Antikörpern inkubiert wurden.

10. Antigene gemäß Anspruch 9, dadurch gekennzeichnet, daß sie von Human-Seren, die Antikörper gegen Leishmanien enthalten, erkannt werden.

11. Antigene gemäß Anspruch 9 oder Anspruch 10, dadurch gekennzeichnet, daß sie ein schützendes Glycoprotein enthalten, das ein Molekulargewicht der Größenordnung von 70 kD aufweist und Glucose- und Mannosegruppen umfaßt.

12. Antigene gemäß Anspruch 9 oder Anspruch 10, dadurch gekennzeichnet, daß sie Glycoproteine enthalten, die aus Lysaten von Leishmanien isolierbar sind, die Molekulargewicht der Größenordnung von 20 kD aufweisen und Fucose- und α-D-N-Acetylgalactosamingruppen enthalten.

13. Antigene gemäß Anspruch 9 oder Anspruch 10, dadurch gekennzeichnet, daß sie Glycoproteine enthalten, die aus Lysaten von Leishmanien isolierbar sind, die Molekulargewicht der Größenordnung von 30—32 kD aufweisen und Fucose- und α-D-N-Acetylgalactosamingruppen enthalten.

14. Komposition zur Herstellung von Impfstoffen für Menschen oder Tiere, gekennzeichnet durch die Verbindung von einem oder mehreren Polypeptiden gemäß einem der Ansprüche 9 bis 13 mit einem pharmazeutischen Transportmittel, das zur Impfung geeignet ist, bevorzugt auf parenteralem Wege.

15. Verfahren zum Nachweis oder der Diagnose der Infektion durch die Leishmanien bei Menchen oder Tieren *in vitro*, dadurch gekennzeichnet, daß es das in Kontakt brigen der Zellen, die insbesondere aus einer Gewebsbiopsie (Knochenmark, Milz oder Leber) stammen, mit monoklonalen Antikörpern, wie sie in einem der Ansprüche 1 bis 5 definiert sind, umfaßt und den Nachweis, insbesondere durch Immunofluoreszenz, von einer Reaktion zwischen den genannten monoklonalen Antikörpern und den genannten Zellen im Falle einer Infektion.

16. Verfahren, das die Antigene gemäß einem der Ansprüche 9 bis 13 verwendet, das erlaubt, *in vitro* das Vorhandensein von Anti-Leishmania-Antikörpern im Serum eines meschlichen oder tierischen Patienten nachzuweisen, und so die Diagnose der Infektion bei Menchen oder Tieren *in vitro* durchzuführen.

## Claims

1. Anti-Leishmania monoclonal antibodies having the essential characteristics of those which are secreted by hybridomes obtained by fusion of myeloma SP2/0 cells and spleen cells removed from Balb-C mice previously infected with promastigotes of the *L. infantum* strain deposited with the C.N.C.M. on September 26, 1984 under the number I-342, the supernatants of the clone cultures or the corresponding ascitic liquids being sorted to retain a first step (on the majority) only the monoclonal antibodies which bind several Leishmanias at a time, advantageously onto at least one of the parasites belonging to each of the species *L. infantum, L. tropica, L. brasiliensis* and *L. mexicana* (detection of the binding by global indirect immunofluorescence) and, at the end of the second sorting, those of the monoclonal antibodies which bind onto the living or killed parasites, said monoclonal antibodies being characterized:

in that they recognize antigens common to various Leishmania species, notably *L. infantum, L. mexicana, L. tropica, L. donovani* and *L. brasiliensis,*

by their capacity to inhibit the infection of sarcomatous cells of mice belonging to the TG180 line, when $2.10^3$ cells of this line are injected into the peritoneum of Balb-C mice, mixed with 60 microliters of ascites containing the aforesaid monoclonal antibodies and $10^5$ promastigotes of one or several Leishmania species which have been previously incubated in the presence of said monoclonal antibodies at 37°C for 30 minutes.

2. Monoclonal antibodies according to Claim 1, characterized in that they are secreted by the hybridomes which have been deposited with the C.N.C.M. respectively under the numbers I-344 and I-345 the 1st of October 1984.

3. Monoclonal antibodies according to Claim 1 or 2, characterized in that they recognize proteins having molecular weights:

of less than 20,000, or

of the order of 30,000,

or of the order of 40,000,

from 67,000 to 94,000, notably of the order of 70,000,

of the order of 113,000,

contained in the lysates of Leishmanias.

4. Monoclonal antibodies according to Claim 1 or 2, characterized in that they recognize proteins having molecular weights of the order of 140,000 and contained in the lysates of Leishmanias.

5. Monoclonal antibodies according to any one of the Claims 1 to 4, characterized in that they recognize the proteins which are equally recognized *in vitro* by sera coming from patients infected with Leishmaniosis.

6. Secreting hybridomes, characterized by the fact that the monoclonal antibodies which they secrete are conform to those defined in any one of the Claims 1 to 5.

7. A process for the obtaining and isolation of secreting hybridomes according to Claim 6 characterized in that one places in contact the infectious promastigote forms of a Leishmania specie with, on the one hand, monoclonal antibodies secreted by hybridomes obtained by fusion of myeloma SP2/0 cells and spleen cells removed from Balb-c mice previously infected with promastigotes of the *L. infantum* strain deposited with the C.N.C.M. on September 26, 1984 under the number I-342, and, on the other hand, with sarcomatous cells under conditions which, in the absence of the said antibodies, would lead to a parasitic infection of the said sarcomatous cells, and in that one selects those of these monoclonal antibodies which, in the preceding operation, completely protect in vivo the sarcomatous cells vis-à-vis infection by the said Leishmanias, after their injection into the peritoneum of a mouse sensitive to said sarcomatous cells, more particularly a Balb-C mouse.

8. Process according to Claim 6, characterized in that the sarcomatous cells utilized belong to the line of mouse sarcomatous cells called TG180.

9. Antigens of the parasites of the leishmanioses having molecular weights of the order either of up to 20,000, or of the order of 40,000, or 70,000 or 113,000, or 140,000 daltons, these polypeptides being characterized:

in that they are recognized by the monoclonal antibodies such as defined in Claim 1, notably the monoclonal antibodies secreted by the hybridomes deposited with the C.N.C.M. under the no I-344 and I-345 the 1st of October 1984,

by their capacity to induce *in vivo* the production of antibodies capable of inhibiting the infection of sarcomatous cells of mice belonging to the TG180 line, when $2.10^3$ cells of this line are injected into the peritoneum of Balb-C mice, mixed with $10^5$ promastigotes of one or several Leishmanias species, which have been possibly previously incubated with the above mentioned antibodies.

10. Antigens according to Claim 9, characterized in that they are recognized by human sera containing antibodies with regard to the Leishmanias.

11. Antigens according to Claim 9 or Claim 10, characterized in that they contain a protective glycoprotein having a molecular weight of the order of 70 kD and including glucose and mannose groups.

12. Antigens according to Claim 9 or Claim 10, characterized in that they contain glycoproteins isolatable from lysates of the Leishmanias presenting molecular weights of the order of 20 kD and containing fucose and alpha-D-N-acetyl-galactosamine groups.

13. Antigens according to Claim 9 or Claim 10, characterized in that they contain glycoproteins isolatable from lysates of the Leishmanias presenting molecular weights of the order of 30—32 kD and containing fucose and alpha-D-N-acetyl-galactosamine groups.

14. Composition for the production of vaccines, for man or animal, characterized by the association of one or more polypeptides according to any one of the Claims 9 to 13, with a vehicle pharmaceutically appropriate to vaccination, preferably parenterally.

15. A process for the detection or diagnosis *in vitro* of the infection by the Leishmanias in man or animal, characterized in that it includes the placing in contact the cells, coming notably from a tissue biopsy (bone marrow, spleen or liver), with monoclonal antibodies such as defined in any one of the Claims 1 to 5, and the detection, notably by immunofluorescence, of a reaction between the said monoclonal antibodies and the said cells in the case of infection.

16. A process using the antigens according to any one of the Claims 9 to 13, permitting the detection *in vitro* of the presence of anti-Leishmania antibodies in the serum of a human or animal patient and to thus effect the diagnosis *in vitro* of the infection in man or animal.